# FASCICULE DE BREVET EUROPEEN

(11) **EP 3 145 580 B1**
(45) Date de publication et mention de la délivrance du brevet: **11.12.2019**
(21) Numéro de dépôt: 15732354.4
(22) Date de dépôt: 11.05.2015
(51) Int. Cl.: A61N 1/04, A61N 1/36

(54) **DISPOSITIF D'ELECTRODE CUTANEE ET DISPOSITIF D'ELECTROSTIMULATION INTEGRANT CE DISPOSITIF D'ELECTRODE**
KUTANE ELEKTRODENVORRICHTUNG UND ELEKTROSTIMULATIONSVORRICHTUNG MIT BESAGTER ELEKTRODENVORRICHTUNG
CUTANEOUS ELECTRODE DEVICE AND ELECTROSTIMULATION DEVICE INCLUDING SAID ELECTRODE DEVICE

(30) Priorité: 19.05.2014 FR 1454462
(43) Date de publication de la demande: 29.03.2017
(73) Titulaire: Commissariat à l'Énergie Atomique et aux Énergies Alternatives, 75015 Paris (FR)
(72) Inventeur: KARST, Nicolas, F-57600 Folkling (FR); PERRAUD, Simon, F-83150 Bandol (FR)
(74) Mandataire: Gevers & Orès
(86) Numéro de dépôt international: PCT/IB2015/053457
(87) Numéro de publication internationale: WO 2015/177677

(56) Documents cités:
- FR-A1- 2 778 108
- US-A1- 2002 038 101
- US-A1- 2009 076 559
- US-A1- 2013 023 816

## Description

L'invention concerne le domaine technique des dispositifs destinés à être fixés sur la peau d'un utilisateur.

Il s'agit notamment de dispositifs médicaux, comme des électrodes cutanées pour la mesure de paramètres physiologiques, des électrodes cutanées pour l'électrostimulation ou encore des générateurs d'impulsions pour l'électrostimulation.

En particulier, les dispositifs d'électrostimulation conventionnels se composent d'un générateur d'impulsions électriques relié à des électrodes cutanées.

Le générateur d'impulsions électriques se présente sous la forme d'un boîtier volumineux et rigide et permet d'envoyer des impulsions électriques calibrées en fréquence et en intensité jusqu'à une zone précise du corps humain par l'intermédiaire d'électrodes cutanées. Afin de pouvoir être au contact d'une multitude de zones de stimulation, les électrodes cutanées sont reliées au boîtier par l'intermédiaire de câbles mesurant plusieurs dizaines de centimètres.

On peut se référer au document US5423874 qui décrit un dispositif du type patch. Il est maintenu sur la peau de l'utilisateur par l'intermédiaire d'un adhésif double face dans lequel deux ouvertures ont préalablement été formées de telle sorte que l'anode et la cathode puissent assurer un contact électrique avec la peau de l'utilisateur. L'anode et la cathode sont également en contact avec les broches du circuit électronique. Un autre exemple de dispositif d'électrostimulation du type patch est décrit dans le document WO 2013/106644, ou dans le document US2002038101. Ce dispositif d'électrostimulation se compose d'une paire d'électrodes flexibles incorporée au niveau de la surface inférieure d'un boîtier rigide, au sein duquel est présent l'électronique d'électrostimuiation.

Ces dispositifs ci-dessus (US5423874, WO2013/106644, US2002038101) sont maintenus en place sur la peau du patient par l'intermédiaire des électrodes adhésives. Ils présentent une distance fixe entre l'anode et la cathode, ce qui limite également les zones de stimulation adressables.

Ainsi, ces dispositifs d'électrostimulation ne sont pas polyvalents et ne peuvent être utilisés que pour certaines zones de stimulation du corps humain.

La demande de brevet US2013/0158627, répond à certains problèmes soulevés précédemment mais présente l'inconvénient de n'être pas réellement modulaire en terme d'utilisation. En effet, le choix de la zone stimulée électriquement est déterminé et n'est pas modulable.

L'invention a pour objet de pallier ces inconvénients en proposant un dispositif d'électrode et un dispositif d'électrostimulation pouvant s'adapter à des zones différentes du corps humain pour assurer leur stimulation.

Ainsi, l'invention concerne un dispositif d'électrode cutanée destiné à être connecté à un générateur d'impulsions électriques comprenant un corps plan en un matériau isolant électrique sur lequel sont formés au moins une électrode, au moins un moyen de connexion au générateur d'impulsions électriques et au moins un élément conducteur reliant électriquement ladite électrode audit moyen de connexion.

Selon l'invention, ladite au moins une électrode débouche sur une face interne dudit corps et ledit au moins un moyen de connexion débouche sur une face externe dudit corps, ledit corps comportant au moins une portion en saillie, ladite portion reliant mécaniquement une électrode et un moyen de connexion et étant destinée à relier électriquement et mécaniquement par repliement ledit dispositif d'électrode audit générateur d'impulsions électriques.

De façon avantageuse, c'est l'ensemble dudit corps qui est flexible.

Au moins une partie de ce corps présente une forme allongée, s'étendant selon un axe longitudinal, et comporte au moins deux portions en saillie. Ces dernières peuvent être disposées de part et d'autre de cet axe.

Ce corps comporte au moins une branche s'étendant depuis la partie allongée dudit corps en faisant un angle non nul avec son axe longitudinal, une électrode étant réalisée sur ladite au moins une branche.

Le dispositif d'électrode peut également comprendre une grille formée sur la face interne dudit corps et reliée électriquement à ladite au moins une électrode.

Dans une variante de réalisation, le dispositif comprend également au moins une portion en saillie complémentaire avec un trou, cette portion s'étendant sensiblement selon le même axe que ladite au moins une portion et du côté opposé par rapport audit corps.

Le dispositif comprend avantageusement une couche d'hydrogel, prévue sur ledit corps au niveau de ladite au moins une électrode.

Le dispositif peut également comprendre un adhésif double face sur les zones du corps dépourvues d'éléments conducteurs.

L'invention concerne également un dispositif d'électrostimulation formé par le dispositif d'électrode selon l'invention et un générateur d'impulsions électriques comprenant au moins un moyen de connexion sur sa face supérieure, la face inférieure dudit générateur d'impulsions électriques étant susceptible de venir en contact avec la face externe dudit dispositif d'électrode, et au moins une portion en saillie étant susceptible de venir en contact avec la face supérieure du générateur d'impulsions électriques par repliement et de le relier mécaniquement et électriquement audit dispositif d'électrode.

De manière avantageuse, le générateur d'impulsions électriques comprend au moins deux zones rigides reliées électriquement et mécaniquement par une zone flexible, ces zones rigides portant les composants du générateur d'impulsions électriques ainsi que les moyens de connexion à des électrodes.

De façon avantageuse, ce dispositif d'électrostimulation comporte des moyens magnétiques au niveau des moyens de connexion électrique prévus sur le dispositif d'électrode ou sur le générateur d'impulsions électriques.

Ce dispositif d'électrostimulation comporte avantageusement au moins une électrode cutanée susceptible d'être reliée électriquement audit au moins un moyen de connexion du générateur d'impulsions électriques.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 est une vue de dessus d'un dispositif d'électrode selon l'invention,
- la figure 2 est une vue de dessous du dispositif d'électrode illustré à la figure 1,
- la figure 3 est une vue en coupe selon la ligne II-II du dispositif illustré à la figure 2,
- la figure 4 est une vue de dessus d'un exemple de générateur d'impulsions électriques pouvant être associé au dispositif d'électrode illustré aux figures 1 à 3,
- la figure 5 est une vue en coupe selon la ligne V-V de la figure 4,
- la figure 6 est une vue de dessus d'un ensemble comprenant un dispositif d'électrode illustré aux figures 1 à 3, sur lequel est disposé un générateur d'impulsions électriques selon les figures 4 et 5,
- la figure 7 est une vue de dessus d'un autre exemple de réalisation de dispositif d'électrode selon l'invention,
- la figure 8 est une vue de dessous du dispositif d'électrode illustré à la figure 7, et
- la figure 9 est une vue de dessus d'un ensemble comprenant un dispositif d'électrode selon les figures 7 et 8, sur lequel est disposé un générateur d'impulsions électriques selon les figures 4 et 5,
- la figure 10 est une vue de dessus d'un autre exemple de réalisation du dispositif d'électrode selon l'invention,
- la figure 11 est une vue de dessus d'une variante de réalisation du dispositif d'électrode illustré aux figures 1 à 3 et
- la figure 12 est une vue de dessus d'un ensemble comprenant un dispositif d'électrode illustré à la figure 11, sur lequel est disposé un générateur d'impulsions électriques selon les figures 4 et 5.

Les éléments communs aux différentes figures seront désignés par les mêmes références.

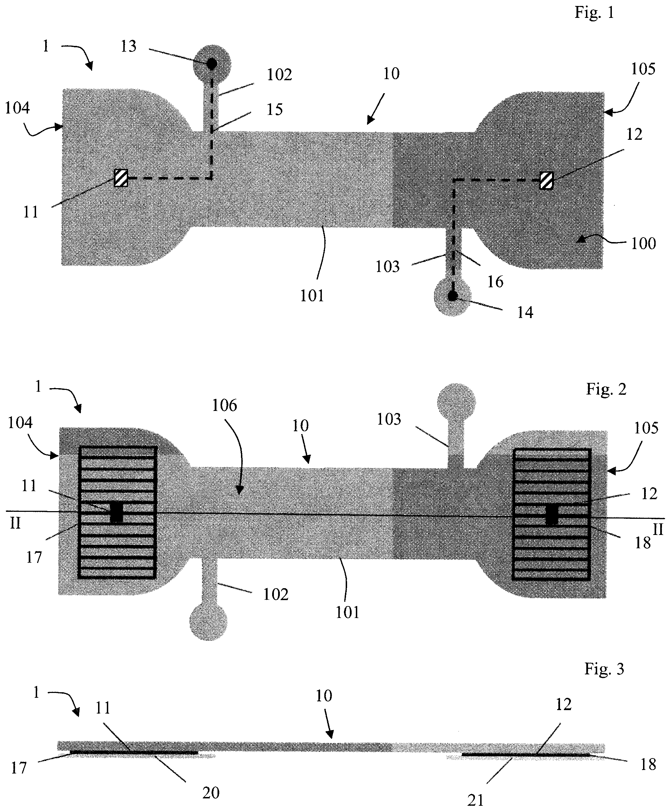

La figure 1 illustre un dispositif d'électrode 1 comportant un corps 10 réalisé en un matériau isolant électrique.

Ce corps 10 sera de préférence flexible et pourra notamment être réalisé à base de silicone.

Ce corps 10 est plan et comporte une partie centrale 101 avec deux portions en saillie 102, 103 qui s'étendent à partir de cette partie centrale 101, ainsi que deux parties latérales 104, 105 de part et d'autre de la partie centrale 101.

Dans l'exemple de réalisation représenté sur les figures 1 et 2, les portions en saillie se présentent chacune sous la forme d'une languette.

Selon un mode particulier de réalisation non représenté, chaque portion en saillie pourrait prendre la forme d'un rabat allongé s'étendant le long du corps 10. De façon générale, les portions en saillie (languettes ou rabats) pourront présenter des formes différentes de celles illustrées à la figure 1.

Dans l'exemple illustré à la figure 1, le corps 10 présente une forme allongée qui s'étend selon un axe longitudinal. Par ailleurs, les portions en saillie sont ici sensiblement perpendiculaires à cet axe longitudinal et elles s'étendent dans des directions opposées.

Dans le corps 10, sont réalisées deux électrodes 11 et 12, deux moyens de connexion tels que des fiches de connexion 13 et 14 ainsi que deux éléments conducteurs 15 et 16.

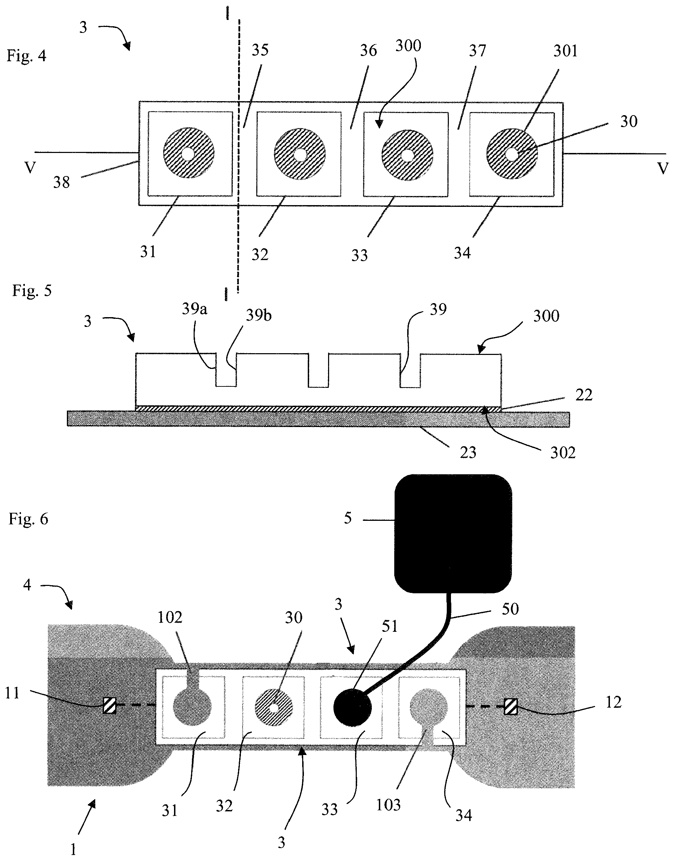

Afin d'assurer une connexion électrique avec les moyens de connexion d'un générateur d'impulsions électriques, les fiches de connexion 13 et 14 sont débouchantes sur une face externe 100 du corps 10. La face externe 100 est destinée à venir en contact à la fois avec une face inférieure et une face supérieure d'un générateur d'impulsions électriques qui sera notamment décrit en référence aux figures 4 et 5.

Afin d'assurer une connexion électrique avec une partie du corps humain, les deux électrodes 11 et 12 sont débouchantes sur une face interne 106 du corps 10, illustrée à la figure 2. La face interne 106 est destinée venir en contact avec le corps humain (ou avec un hydrogel qui lui sera en contact avec le corps humain).

La figure 1 montre que les fiches de connexion 13 et 14, destinées à assurer la connexion électrique entre chaque électrode et un moyen de connexion 30 correspondant d'un générateur d'impulsions électriques, sont chacune disposées sur une portion en saillie 102, 103. Elles sont, de préférence, situées au niveau de l'extrémité libre de ces portions en saillie.

Par ailleurs, les deux éléments conducteurs 15 et 16 sont formés dans le corps 10. Le premier élément conducteur 15 permet de relier électriquement une première électrode 11 à une première fiche de connexion 13, tandis que le second élément conducteur 16 permet de relier électriquement une seconde électrode 12 à une seconde fiche de connexion 14.

De plus, chaque électrode est reliée mécaniquement à une fiche de connexion par l'intermédiaire d'une portion en saillie.

Les éléments conducteurs 15 et 16 pourront se présenter sous la forme d'un simple câble conducteur électrique ou d'un élément conducteur de type piste métallique, reportée à la surface du corps 10 et de la portion en saillie 102 ou 103.

Pour des raisons de sécurité, chaque élément conducteur 15 ou 16 pourra être totalement ou partiellement encapsulé au sein du corps 10 et de la portion en saillie 102 ou 103. De même, les électrodes 11 et 12 présentes au niveau de la face externe 100 pourront être encapsulées au sein du corps 10 au niveau de la face externe du corps et apparentes sur sa face interne. Les fiches de connexion 13 et 14 sont conçues de façon à coopérer avec les moyens de connexion présents sur le générateur d'impulsions électriques associé au dispositif d'électrode et dont un exemple est décrit en référence aux figures 4 et 5.

Les portions en saillie 102 et 103 pourront être disposées de part et d'autre du corps 10 comme illustré sur la figure 1 mais pourront également être disposées du même côté, selon l'axe longitudinal du corps 10.

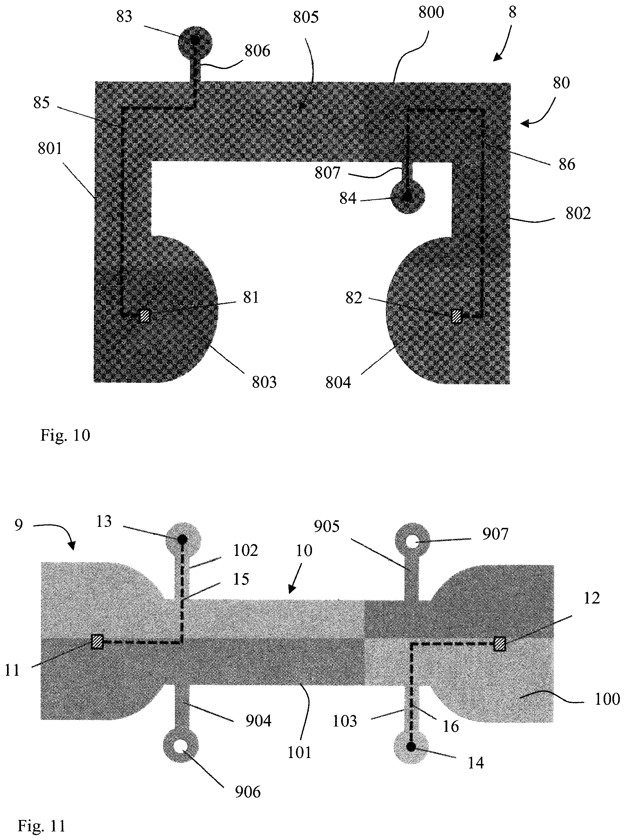

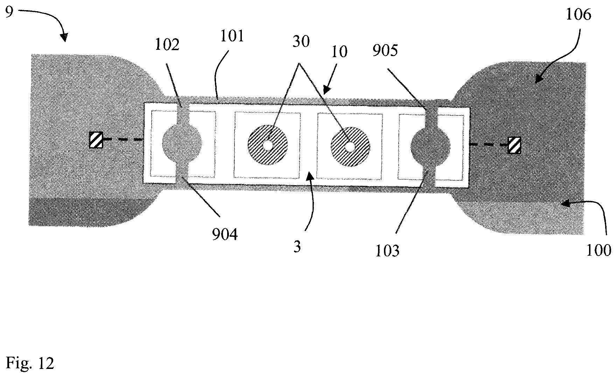

Lorsque les portions en saillie 102 et 103 sont disposées de part et d'autre du corps 10, le maintien du générateur d'impulsions électriques peut être amélioré, comme cela sera décrit en référence à la figure 6. De plus, afin d'améliorer davantage le maintien du générateur d'impulsions électriques, un autre mode de réalisation d'un dispositif d'électrode cutané sera présenté sur la figure 11 et 12.

Il est maintenant fait référence aux figures 2 et 3 qui représentent respectivement la face interne 106 du dispositif d'électrode 1 et une vue en coupe selon la ligne II-II.

Ces deux figures illustrent une grille 17 ou 18 qui peut être prévue au niveau de l'électrode 11 ou 12, afin de diffuser de façon homogène le courant au niveau de la peau de l'utilisateur.

La surface occupée par chacune des grilles 17 ou 18 sera comprise entre 4 cm² et 150 cm² et de préférence égale à 50 cm². La surface occupée par la grille sera avantageusement supérieure à 5% de la surface du corps 10.

De plus, afin d'améliorer le transfert du courant entre les électrodes 11 ou 12 et la peau de l'utilisateur, une couche d'hydrogel 20 ou 21 peut être ajoutée. Cette couche d'hydrogel 20 ou 21 peut, en plus de sa propriété de conduction du courant, avoir une propriété d'adhésion (adhésion, à la fois sur le dispositif d'électrode 1 et sur la peau).

Afin de renforcer le maintien du dispositif d'électrode 1 sur la peau de l'utilisateur, un adhésif pourra être prévu sur la face interne 106 du corps 10, opposée à la face 100. D'autres moyens de fixation peuvent être prévus comme un bracelet, une jambière, un brassard ou encore une ceinture.

L'invention n'est pas limitée à ce mode de réalisation. En particulier, le corps 10 pourrait ne comporter qu'une électrode ou une pluralité d'électrodes.

Il est maintenant fait référence aux figures 4 et 5 qui illustrent un exemple de générateur d'impulsions électriques qui peut être utilisé en combinaison avec le dispositif d'électrode décrit en référence aux figures 1 à 3 pour former un dispositif d'électrostimulation.

Ce générateur d'impulsions électriques 3 présente ici une forme rectangulaire et présente un ou plusieurs axes de flexion parallèles à l'axe I-I. Dans l'exemple illustré sur la figure 3, le générateur d'impulsions électriques 3 est composé de quatre zones rigides 31, 32, 33, 34 reliées électriquement et mécaniquement par trois zones flexibles 35, 36, 37 s'étendant selon l'axe I-I ou un axe parallèle à l'axe I-I.

Plus généralement, le générateur d'impulsions électriques 3 pourra être composé d'au moins deux zones rigides reliées électriquement et mécaniquement par au moins une zone flexible, permettant une flexion selon au moins un axe parallèle à l'axe I-I.

Chacune des zones rigides permet d'accueillir un ou plusieurs composants du générateur d'impulsions électriques 3. On pourra, par exemple, disposer l'électronique d'électrostimulation sur la zone rigide 31 et trois batteries sur les zones rigides 32, 33 et 34.

Cet exemple de générateur d'impulsions électriques est flexible. Dans d'autres exemples de réalisation, le générateur d'impulsions électriques pourrait être flexible sur l'ensemble de sa surface, à condition que tous les éléments composant le générateur d'impulsions électriques soient également flexibles.

Afin de protéger les composants électroniques et les batteries du milieu extérieur, ils sont avantageusement encapsulés par un revêtement 38. Ce revêtement 38 pourra être un élément en silicone surmoulé épousant les formes particulières du générateur d'impulsions électriques.

L'épaisseur de cet élément 38 sera comprise entre 0.2 mm et 5 mm. Il pourra être présent à la fois sur le dessus et le dessous du générateur d'impulsions électriques 3. Ce revêtement 38 pourra recouvrir partiellement ou totalement les éléments rigides et les éléments flexibles. La surface de ce revêtement 38 pourra être supérieure à la surface cumulée des éléments flexibles et des éléments rigides.

Comme illustré sur la figure 5, le générateur d'impulsions électriques 3 pourra présenter des renfoncements 39 afin d'améliorer sa flexibilité.

La profondeur des renfoncements sera préférentiellement supérieure à 50% de l'épaisseur totale du générateur d'impulsions électriques 3.

Sur chacune des zones rigides 31, 32, 33 et 34, est prévu un moyen de connexion 30 permettant de connecter électriquement une électrode cutanée au générateur d'impulsions électriques.

Dans un mode avantageux de réalisation du générateur 3, chacun des moyens de connexion électrique 30 peut être conçu pour définir une zone rigide du générateur et ainsi assurer une protection mécanique efficace des composants présents dans cette zone.

Ainsi, le générateur peut comprendre deux couches successives, à savoir une première couche comprenant les moyens de connexion électrique et une deuxième couche comprenant les composants sensibles à la flexion.

Chacun des moyens de connexion électrique présente alors, dans le plan du dispositif, une surface correspondant à au moins un composant et est disposé de façon à recouvrir ce composant. Il définit ainsi une zone rigide dans laquelle la protection mécanique de ce composant est assurée.

De façon à assurer la protection mécanique de l'ensemble des composants présents dans une portion du générateur 3, chacun des moyens de connexion présente, dans le plan du dispositif, une surface supérieure à celle occupée par ces composants et est aligné avec eux selon l'épaisseur de cette portion du générateur, de manière à les recouvrir.

Grâce à ce mode de réalisation, il n'est pas nécessaire de prévoir un boîtier rigide pour protéger les composants de contraintes mécaniques.

De plus, dans la mesure où des composants sensibles à la flexion peuvent être présents dans les zones flexibles 35, 36, 37 , le générateur 3 peut également comprendre des moyens de limitation de la flexion pour limiter ou bloquer la flexion de ces zones flexibles par rapport à un axe parallèle à l'axe I-I.

Dans l'exemple illustré à la figure 5, les parois en regard 39a et 39b de deux zones rigides adjacentes peuvent constituer des moyens de limitation de la flexion par rapport à un axe parallèle à l'axe I-I, lorsque cette flexion tend à rapprocher l'une de l'autre ces parois en regard.

Les moyens de connexion 30 sont situés sur la face supérieure 300 du générateur d'impulsions électriques 3 et se présentent ici sous la forme de trous.

Dans le cas où les moyens de connexion 30 sont des trous, les fiches de connexion du dispositif d'électrode 1 pourront être des éléments partiellement métalliques présentant une partie en saillie permettant de coopérer avec ces moyens de connexion 30. La coopération entre le dispositif d'électrode et le générateur d'impulsions électriques 3 est décrite en référence à la figure 6.

Par ailleurs, différents moyens de connexion peuvent être envisagés pour connecter électriquement une électrode au générateur d'impulsions électriques. Ces moyens peuvent comprendre une fiche reliée électriquement à l'électrode par l'intermédiaire d'un câble, la fiche étant conçue pour coopérer avec au moins un moyen de connexion présent sur le générateur d'impulsions électriques, de façon à connecter électriquement cette fiche au circuit électronique du générateur d'impulsions électriques.

Par exemple, la fiche et le moyen de connexion peuvent former un système de type bouton pression déjà utilisé dans certains dispositifs d'électrostimulation conventionnels pour connecter les câbles provenant du boîtier aux électrodes cutanées.

Sur le générateur d'impulsions électriques 3 illustré sur la figure 4, il est possible de connecter simultanément jusqu'à quatre électrodes cutanées par l'intermédiaire des quatre moyens de connexion 30.

Le revêtement 38 présentera des ouvertures au niveau des trous 30, afin que les fiches puissent être connectées électriquement au circuit électronique.

Afin de faciliter la connexion des électrodes cutanées par l'intermédiaire des fiches, des éléments magnétiques pourront être intégrés au niveau des moyens de connexion 30 du générateur d'impulsions électriques 3 et au niveau des fiches.

Ces éléments magnétiques sont destinés à exercer l'un sur l'autre une force magnétique attractive. Ainsi, il suffit qu'une fiche soit proche d'un moyen de connexion pour que les forces magnétiques entrent en action et que la connexion entre la fiche et le moyen de connexion soit réalisée.

Dans l'exemple illustré sur la figure 4, des éléments magnétiques 301 sont intégrés au niveau du générateur d'impulsions électriques.

Les éléments 301 peuvent être des aimants à base de néodyme, fer et bore.

Les éléments 301 pourront être recouverts par le revêtement 38.

Afin de maintenir le générateur d'impulsions électriques 3 sur la peau de l'utilisateur, un adhésif double face 22 peut être utilisé comme illustré à la figure 5. Une face de l'adhésif 22 est alors en contact avec la face inférieure 302 du générateur d'impulsions électriques 3, une autre face de l'adhésif 22 étant en contact avec la peau de l'utilisateur 23. Ainsi, la face inférieure 302 du générateur d'impulsions électriques 3, opposée à la face supérieure 300, est destinée à être en regard de la peau de l'utilisateur.

Cependant, le générateur d'impulsions électriques décrit aux figures 4 et 5 est, de préférence, utilisé avec le dispositif d'électrode décrit en référence aux figures 1 à 3.

Il est maintenant fait référence à la figure 6 qui illustre la mise en place du générateur d'impulsions électriques 3 sur le dispositif d'électrode 1.

La figure 6 montre que le générateur d'impulsions électriques 3 est disposé sur la partie centrale 101 du corps 10 et sur la face externe 100 dudit corps 10, face sur laquelle débouchent les fiches de connexion 13 et 14. La face inférieure 302 du générateur d'impulsions électriques 3 se trouve ainsi en contact avec la face externe 100 du corps 10 du dispositif d'électrode, au niveau de sa partie centrale 101.

La figure 6 montre qu'après la mise en place du générateur d'impulsions électriques 3, les portions en saillie 102 et 103 sont repliées sur la face supérieure du générateur d'impulsions électriques 3 de façon à ce que les fiches de connexion 13 et 14 soient connectées électriquement à un moyen de connexion 30. Ainsi, une partie de la face externe 100 du corps 10, située au niveau des portions en saillie 102 et 103, se retrouve en contact avec la face supérieure 300 du générateur d'impulsions électriques 3.

La connexion électrique entre l'une des électrodes 11 et 12 du dispositif d'électrode 1 et un moyen de connexion 30 du générateur d'impulsions électriques 3 est donc réalisée sur la face supérieure 300 du générateur d'impulsions électriques 3. Cette configuration permet de connecter d'autres électrodes cutanées sur le dispositif d'électrostimulation formé par le dispositif 1 et le générateur d'impulsions électriques 3, comme cela sera décrit plus loin et contribue à la polyvalence de l'ensemble.

Ainsi, une fois les fiches de connexion 13 et 14 connectées, les portions en saillie 102 et 103 permettent, en plus de la connexion électrique, de maintenir de façon solidaire et amovible le générateur d'impulsions électriques 3 au dispositif d'électrode pour former un dispositif d'électrostimulation.

Le dispositif d'électrostimulation 4 obtenu est ensuite appliqué sur la peau du patient et il est maintenu en place grâce aux couches d'hydrogel 20 et 21 présent au niveau des électrodes 11 et 12 et/ou grâce à l'adhésif double face 22.

Ainsi, grâce à la configuration de l'invention, la face supérieure et notamment les moyens de connexion 30 du générateur d'impulsions électriques sont accessibles.

Il est alors également possible de connecter une électrode cutanée supplémentaire au niveau des moyens de connexion 30 des zones rigides 32 et 33. Une telle électrode 5 est illustrée sur la figure 6, connectée à la zone 33 par l'intermédiaire du câble 50 et du moyen de connexion 51.

Il convient de noter qu'au moins un des moyens de connexion 13 ou 14 pourrait ne pas être relié électriquement aux moyens de connexion 30 présents sur le générateur d'impulsions électriques, Dans ce cas, la(les) languette(s) 102 et/ou 103 n'assure(nt) que le maintien mécanique du générateur d'impulsions électriques 3 sur le dispositif d'électrode 1.

Grâce au dispositif d'électrode, le générateur d'impulsions électriques 3 pourra être utilisé pour traiter des pathologies comme les lombalgies, en le plaçant au niveau du bas du dos.

De plus, du fait de l'architecture spécifique du dispositif d'électrostimulation (patch), il est possible, comme évoqué précédemment, de coupler le dispositif d'électrode à deux autres électrodes cutanées permettant ainsi avec un même dispositif d'électrostimulation de réaliser simultanément des stimulations antalgiques (inhibant instantanément la douleur), délivrées par l'intermédiaire du dispositif d'électrode 1, et des stimulations endorphiniques (prolongeant l'absence de sensations douloureuses du fait de la production d'endorphine), délivrées par l'intermédiaire des électrodes cutanées 5.

Enfin, une fois retiré du dispositif d'électrode, le générateur d'impulsions électriques 3 pourra être utilisé pour traiter d'autres endroits du corps humain, en utilisant notamment des électrodes cutanées 5 comme représenté à la figure 6.

Ainsi, grâce au dispositif d'électrode, le dispositif d'électrostimulation selon l'invention présente une polyvalence permettant d'être utilisé par des patients présentant des pathologies variées mais également par des patients avec des multi-pathologies. Il est également possible d'envisager l'utilisation simultanée de plusieurs générateurs d'impulsions électriques à différents endroits du corps humain.

Le dispositif d'électrode selon l'invention pourra s'adapter à d'autres formes de générateurs d'impulsions électriques que celle décrite en référence aux figures 4 et 5.

La surface occupée par le dispositif d'électrode sera préférentiellement au moins égale à la surface occupée par le générateur d'impulsions électriques 3.

Différentes tailles ou formes du dispositif d'électrode pourront être envisagées, en fonction des zones à stimuler ou de la morphologie des personnes utilisant le dispositif d'électrostimulation.

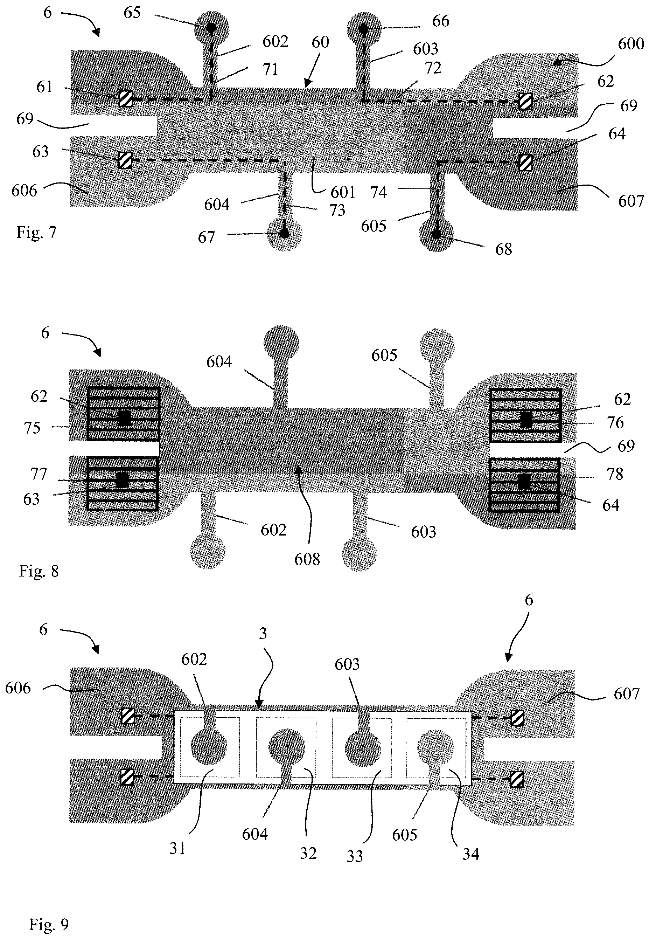

Il est maintenant fait référence aux figures 7 à 9 qui illustrent une variante de réalisation du dispositif d'électrode selon l'invention.

Dans cette variante de réalisation, à partir de la partie centrale 601 du corps 60, s'étendent quatre portions en saillie 602, 603, 604 et 605, ici sous la forme d'une languette.

Le corps 60 comporte, de part et d'autre de la partie centrale 601, deux parties latérales 606 et 607.

La figure 7 montre que les languettes 602 et 603 s'étendent du même côté de l'axe longitudinal selon lequel s'étend le corps 60, tandis que les languettes 604 et 605 s'étendent de l'autre côté de cet axe par rapport aux languettes 602 et 603.

Dans l'exemple illustré sur les figures 7 à 9, ces languettes s'étendent sensiblement perpendiculairement à l'axe longitudinal du corps 60.

Par ailleurs, les languettes 602 à 605 sont disposées de façon alternée de part et d'autre de la partie centrale 601 du corps 60. L'intérêt de cette disposition sera illustré par la figure 8.

Dans le corps 60, sont réalisées quatre électrodes 61, 62, 63 et 64, quatre fiches de connexion 65, 66, 67 et 68 ainsi que des éléments conducteurs 71, 72, 73 et 74.

Afin d'assurer une connexion électrique avec les moyens de connexion 30 d'un générateur d'impulsions électriques 3, les fiches de connexion 65, 66, 67 et 68 sont débouchantes sur une face externe 600 du corps 60. La face externe 600 est destinée à venir en contact avec la face inférieure 302 d'un générateur d'impulsions électriques 3, au niveau de la partie centrale du corps 60.

Afin d'assurer une connexion électrique avec une partie du corps humain, les quatre électrodes 61, 62, 63 et 64 sont débouchantes sur une face interne 608 du corps 60. Cette face 608 est opposée à la face externe 600 et elle est destinée à venir en contact avec le corps humain.

Ces éléments conducteurs 71 à 74 permettent de relier électriquement chaque électrode 61 à 64 à un moyen de connexion 65 à 68, par exemple des fiches de connexion.

Par ailleurs, chaque électrode est reliée mécaniquement à une fiche de connexion par l'intermédiaire d'une languette.

Chacune de ces languettes est de préférence flexible.

Dans l'exemple illustré à la figure 7, les éléments conducteurs 71 à 74 se présentent sous la forme d'une piste métallique formée sur la face externe 600 du corps 60.

Dans une autre forme de réalisation, ces éléments conducteurs pourraient se présenter sous la forme d'un câble conducteur électrique.

Comme décrit en référence à la figure 1, pour des raisons de sécurité, les éléments conducteurs 61 à 64 peuvent être totalement ou partiellement encapsulés au sein du corps 60 et de chacune des languettes 602 à 605.

Les moyens de connexion 65 à 68 sont conçus de façon à coopérer avec les moyens de connexion présents sur un générateur d'impulsions électriques tel que celui illustré sur les figures 4 et 5.

Une grille 75, 76, 77, 78 peut être prévue sur la face interne 608 du corps 10 opposée à la face externe 600 et au niveau de l'électrode 61, 62, 63 ou 64, afin de diffuser de façon homogène le courant au niveau de la peau de l'utilisateur.

La surface occupée par chacune des grilles 75 à 78 sera comprise entre 4 cm² et 150 cm² et préférentiellement égale à 50 cm².

De même, afin d'améliorer le transfert du courant entre les électrodes 61 à 64 et la peau de l'utilisateur, une couche d'hydrogel peut être ajoutée (non représentée sur les figures).

On pourra également envisager d'utiliser quatre éléments distincts à base d'hydrogel, chaque élément d'hydrogel étant mis en regard de l'une des grilles 75 à 78, pour améliorer le transfert du courant entre les électrodes et la peau de l'utilisateur.

Compte tenu de la forte résistivité latérale des éléments d'hydrogel, on pourra également envisager d'utiliser uniquement deux éléments d'hydrogel, l'un étant en regard des grilles 75 et 76, l'autre étant en regard des grilles 77 et 78.

Afin de renforcer le maintien du générateur d'impulsions électriques3 sur la peau, on pourra ajouter un adhésif en face arrière de la partie centrale du corps 60.

Le corps 60 du dispositif d'électrode 6 présente un évidement 69 dans chaque partie latérale 606, 607 du corps 60, de façon à améliorer la conformabilité du dispositif d'électrode. D'autres formes de ce corps 60 pourraient également être envisagées.

Il est maintenant fait référence à la figure 9 qui illustre le générateur d'impulsions électriques 3 après sa mise en place sur le dispositif d'électrode 6.

Cette mise en place s'effectue comme précédemment décrit en référence à la figure 6, pour le dispositif d'électrode 4.

La figure 9 montre qu'après la mise en place du générateur d'impulsions électriques 3, les portions en saillie 602, 603, 604 et 605 sont repliées sur la face supérieure 300 du générateur d'impulsions électriques 3, de façon à ce que les fiches de connexion 65, 66, 67 et 68 soient connectées électriquement au moyen de connexion 30. Ainsi, une partie de la face externe 600 du corps 60, située au niveau des portions en saillie 602, 603, 604 et 605, se retrouve en contact avec la face supérieure 300 du générateur d'impulsions électriques 3.

La figure 9 montre que les moyens de connexion 65 à 68 sont connectés électriquement aux moyens de connexion 30 de chacune des zones rigides 31 à 34 du générateur d'impulsions électriques 3, grâce au repliement de chacune des languettes 602 à 605.

Ainsi, une fois les fiches de connexion 65, 66, 67 et 68 connectées, les portions en saillie 602, 603, 604 et 605 permettent, en plus de la connexion électrique, de maintenir de façon solidaire et amovible le générateur d'impulsions électriques 3 au dispositif d'électrode.

Un ou plusieurs moyens de connexion 65 à 68 peuvent ne pas être reliés électriquement aux moyens de connexion 30 présents sur le générateur d'impulsions électriques. Ainsi, les languettes disposant de telles fiches de connexion n'assurent que le maintien mécanique.

Il est maintenant fait référence à la figure 10 qui illustre une variante de réalisation du dispositif d'électrode selon l'invention.

Dans cette variante de réalisation, le dispositif 8 comporte un corps 80 présentant sensiblement une forme de U, avec une partie centrale 800 formant la base du U s'étendant selon un axe longitudinal et deux branches 801 et 802, s'étendant sensiblement perpendiculairement à cet axe longitudinal.

On peut également envisager d'autres formes de réalisation dans lesquelles les deux branches s'étendent dans des directions opposées, le corps ne comporte qu'une branche s'étendant depuis la partie centrale du corps, ou encore la ou les branche(s) s'étende(nt) dans une direction faisant un angle non nul et inférieur à 90° avec l'axe longitudinal de la partie centrale.

Les deux branches 801 et 802 comportent, au niveau de leurs extrémités, une partie plus large 803 et 804.

Dans le corps 80, sont réalisés deux électrodes 81 et 82, deux moyens de connexion tels que des fiches de connexion 83 et 84 ainsi que deux éléments conducteurs 85 et 86.

Afin d'assurer une connexion électrique avec, par exemple, les moyens de connexion 30 du générateur d'impulsions électriques illustré aux figures 4 et 5, les fiches de connexion 83 et 84 sont débouchantes sur une face externe 805 du corps 80.

Par ailleurs, afin d'assurer une connexion électrique avec une partie du corps humain, les deux électrodes 81 et 82 sont débouchantes sur une face interne du corps 80, opposée à la face externe 805. Cette face interne est destinée à venir en contact avec une partie du corps humain (ou avec un hydrogel qui lui sera en contact avec le corps humain).

Les deux éléments conducteurs 85 et 86 sont formés dans le corps 80, le premier élément conducteur 85 permet de relier électriquement la première électrode 81 à la première fiche de connexion 83, tandis que le second élément conducteur 86 permet de relier électriquement la seconde électrode 82 à la seconde fiche de connexion 84.

Les fiches de connexion 83 et 84 sont disposées sur des portions en saillie 806 et 807 qui s'étendent chacune à partir de la base 800 du corps 80.

Ces deux portions en saillie 806 et 807 s'étendent dans des directions opposées, par rapport à la base 800 du corps 80.

Comme expliqué précédemment, notamment au regard des figures 6 et 9, un générateur d'impulsions électriques 3 peut être associé au dispositif d'électrode 8, de telle sorte que sa face inférieure 302 vienne en contact avec la face externe 805 du dispositif 8, au niveau de sa base 800.

Par ailleurs, au niveau des fiches de connexion 83 et 84, la face externe du dispositif 8 vient en contact avec la face supérieure 300 du générateur.

Le générateur d'impulsions électriques peut ainsi être maintenu de façon solidaire et néanmoins amovible sur le dispositif d'électrode 8.

La forme particulière du corps 8 permet d'éloigner les électrodes 81 et 82 du générateur d'impulsions électriques 3. Cet éloignement dépend de la longueur des branches 801 et 802 du corps 80. Les branches sont situées du même côté ou de part et d'autre du corps 80.

Ce dispositif d'électrode pourra avantageusement être utilisé pour traiter des zones douloureuses au niveau d'une articulation et notamment au niveau du genou.

Il est maintenant fait référence aux figures 11 et 12 qui illustrent une variante de réalisation du dispositif d'électrode précédemment décrit en référence aux figures 1 à 3.

Ce dispositif d'électrode 9 comporte les mêmes éléments que le dispositif 1 illustré aux figures 1 à 3. Ces éléments ne seront pas décrits de nouveau.

Ce dispositif 9 comporte également deux parties en saillie complémentaires 904 et 905 qui sont situées en vis-à-vis d'une portion en saillie 102, 103, par rapport à la partie centrale 101 du corps 10.

Ainsi, la portion en saillie 904 s'étend sensiblement selon le même axe que la portion en saillie 102, mais dans une direction opposée par rapport à la partie centrale 101. Il en est de même pour la portion en saillie 905 par rapport à la portion en saillie 103.

Chacune de ces portions en saillie 904, 905 présente un trou traversant 906, 907, dont la fonction va maintenant être expliquée au regard de la figure 12.

Cette figure 12 illustre la mise en place du générateur d'impulsions électriques 3 sur le dispositif d'électrode 9.

Comme expliqué au regard de la figure 6, le générateur d'impulsions électriques 3 est disposé sur la partie centrale 101 du corps 10 et en contact avec la face externe 100 de ce corps 10.

Après la mise en place du générateur 3, les portions en saillie complémentaires 904 et 905 sont repliées sur la face supérieure du générateur d'impulsions électriques 3, en alignant les trous 906 et 907 avec les moyens de connexion 30 du générateur 3.

Les portions en saillie 102 et 103 sont, dans un deuxième temps, repliées également sur la face supérieure du générateur 3, de façon à ce que les fiches de connexion 13 et 14 soient connectées électriquement à un moyen de connexion 30.

Ainsi, les fiches de connexion 13 et 14 passent au travers des trous 906 et 907 pour assurer la connexion électrique avec les moyens de connexion 30.

Cette variante de réalisation permet d'améliorer le maintien mécanique du générateur d'impulsions électriques 3 sur le dispositif d'électrode 9, grâce à la présence des portions en saillie complémentaires 904 et 905.

La description qui précède montre que grâce, au dispositif d'électrode selon l'invention, un dispositif d'électrostimulation peut être adapté à des zones différentes du corps humain, sans modifier sa structure.

Les signes de référence insérés après les caractéristiques techniques figurant dans les revendications ont pour seul but de faciliter la compréhension de ces dernières et ne sauraient en limiter la portée.

## Revendications

1. Dispositif d'électrode cutanée destiné à être connecté à un générateur d'impulsions électriques (3) et comportant :
un corps (10, 60, 80) plan en un matériau isolant électrique sur lequel sont formés au moins une électrode (11, 12 ; 61, 62, 63, 64 ; 81, 82), au moins un moyen de connexion (13, 14 ; 65, 66, 67, 68 ; 83, 84) au générateur d'impulsions électriques (3), et au moins un élément conducteur (15, 16 ; 71, 72, 73, 74 ; 85, 86) reliant électriquement ladite électrode audit moyen de connexion, ladite au moins une électrode débouchant sur une face interne (106, 608) dudit corps et ledit au moins un moyen de connexion débouchant sur une face externe (100, 600, 805) dudit corps et **caractérisé en ce qu'**au moins une partie dudit corps présente une forme allongée, s'étendant selon un axe longitudinal, et comporte au moins deux portions (102, 103 ; 602, 603, 604, 605 ; 806, 807) en saillie, lesdites au moins deux portions reliant mécaniquement une électrode (11, 12 ; 61, 62, 63, 64 ; 83, 84) et un moyen de connexion (13, 14 ; 65, 66, 67, 68 ; 83, 84) et étant destinées à relier mécaniquement et électriquement par repliement ledit dispositif d'électrode audit générateur d'impulsions électriques.

2. Dispositif selon la revendication 1, dans lequel l'ensemble dudit corps est flexible.

3. Dispositif selon la revendication 1 ou 2, dans lequel lesdites au moins deux portions sont disposées de part et d'autre dudit axe longitudinal.

4. Dispositif selon la revendication 1 ou 3, dans lequel ledit corps (8) comporte au moins une branche (801, 802) s'étendant depuis la partie allongée dudit corps en faisant un angle non nul avec son axe longitudinal, une électrode (81, 82) étant réalisée sur ladite au moins une branche.

5. Dispositif selon l'une des revendications 1 à 4, comprenant une grille (17, 18 ; 75, 76, 77, 78) formée sur la face interne dudit corps (10, 60) et reliée électriquement à ladite au moins une électrode (11, 12 ; 61, 62, 63, 64).

6. Dispositif selon l'une des revendications 1 à 5, comprenant au moins une portion en saillie complémentaire (904, 905) avec un trou (906, 907), cette portion s'étendant sensiblement selon le même axe que ladite au moins une portion (102, 103) et du côté opposé par rapport audit corps (10).

7. Dispositif d'électrostimulation formé par un dispositif d'électrode cutanée (1, 6, 8) selon l'une des revendications 1 à 6 et un générateur d'impulsions électriques (3) comportant au moins un moyen de connexion (30) sur sa face supérieure (300), la face inférieure (302) dudit générateur d'impulsions électriques (3) étant susceptible de venir en contact avec la face externe (100, 600) dudit dispositif d'électrode (1, 6) et au moins une portion (102, 103 ; 602, 603, 604, 605 ; 806, 807) en saillie du dispositif d'électrode étant susceptible de venir en contact avec la face supérieure (300) du générateur d'impulsions électriques par repliement et de le relier mécaniquement et électriquement audit dispositif d'électrode.

8. Dispositif d'électrostimulation selon la revendication 7, dans lequel ledit générateur d'impulsions électriques (3) comprend au moins deux zones rigides (31, 32, 33, 34) reliées électriquement et mécaniquement par une zone flexible (35, 36, 37), ces zones rigides accueillant des composants du générateur d'impulsions électriques ainsi que les moyens de connexion (30) à des électrodes.

9. Dispositif d'électrostimulation selon la revendication 7 ou 8, comportant des moyens magnétiques au niveau des moyens de connexion électrique prévus sur le dispositif d'électrode cutanée (1, 6, 8) ou sur le générateur d'impulsions électriques (3).

10. Dispositif d'électrostimulation selon l'une des revendications 7 à 9, comportant une électrode cutanée (5) susceptible d'être reliée électriquement audit au moins un moyen de connexion (30) du générateur d'impulsions électriques.

## Patentansprüche

1. Kutane Elektrodenvorrichtung, die dazu bestimmt ist, an einen elektrischen Impulsgenerator (3) angeschlossen zu werden und umfassend:
- einen ebenen Körper (10, 60, 80) aus einem elektrisch isolierenden Material, auf dem mindestens eine Elektrode (11, 12; 61, 62, 63, 64; 81, 82), mindestens ein Anschlussmittel (13, 14; 65, 66, 67, 68; 83, 84) an den elektrischen Impulsgenerator (3) und mindestens ein leitendes Element (15, 16; 71, 72, 73, 74; 85, 86), das die Elektrode mit dem Anschlussmittel elektrisch verbindet, geformt sind, wobei die mindestens eine Elektrode in eine Innenseite (106, 608) des Körpers mündet und das mindestens eine Anschlussmittel in eine Außenseite (100, 600, 805) des Körpers mündet und **dadurch gekennzeichnet, dass** mindestens ein Teil des Körpers eine längliche Form aufweist, die sich gemäß einer Längsachse erstreckt, und mindestens zwei vorragende Abschnitte (102, 103; 602; 603, 604, 605; 806, 807) umfasst, wobei die mindestens zwei Abschnitte eine Elektrode (11, 12; 61, 62, 63, 64; 83, 84) und ein Anschlussmittel (13, 14; 65, 66, 67, 68; 83, 84) mechanisch verbinden und dazu bestimmt sind, die Elektrodenvorrichtung mit dem elektrischen Impulsgenerator mechanisch und elektrisch durch Überlappung zu verbinden.

2. Vorrichtung nach Anspruch 1, wobei die Gesamtheit des Körpers flexibel ist.

3. Vorrichtung nach Anspruch 1 oder 2, wobei die mindestens zwei Abschnitte beiderseits der Längsachse angeordnet sind.

4. Vorrichtung nach Anspruch 1 oder 3, wobei der Körper (8) mindestens einen Zweig (801, 802) umfasst, der sich ausgehend vom länglichen Teil des Körpers unter Bilden eines Winkels ungleich null mit dessen Längsachse erstreckt, wobei eine Elektrode (81, 82) auf dem mindestens einen Zweig umgesetzt ist.

5. Vorrichtung nach einem der Ansprüche 1 bis 4, umfassend ein Raster (17, 18; 75, 76, 77, 78), das auf der Innenseite des Körpers (10, 60) geformt ist und mit der mindestens einen Elektrode (11, 12; 61, 62, 63, 64) elektrisch verbunden ist.

6. Vorrichtung nach einem der Ansprüche 1 bis 5, umfassend mindestens einen zu einer Öffnung (906, 907) komplementären vorragenden Abschnitt (904, 905), wobei dieser Abschnitt sich im Wesentlichen gemäß derselben Achse wie der mindestens eine Abschnitt (102, 103) und auf der in Bezug auf den Körper (10) gegenüberliegenden Seite erstreckt.

7. Elektrostimulationsvorrichtung, die durch eine kutane Elektrodenvorrichtung (1, 6, 8) nach einem der Ansprüche 1 bis 6 und einen elektrischen Impulsgenerator (3), der mindestens ein Anschlussmittel (30) auf seiner Oberseite (300) umfasst, geformt ist, wobei die Unterseite (302) des elektrischen Impulsgenerators (3) geeignet ist, um mit der Außenseite (100, 600) der Elektrodenvorrichtung (1, 6) in Kontakt zu treten, und mindestens ein vorragender Abschnitt (102, 103; 602, 603, 604, 605; 806, 807) der Elektrodenvorrichtung geeignet ist, um mit der Oberseite (300) des elektrischen Impulsgenerators durch Überlappung in Kontakt zu treten und ihn mit der Elektrodenvorrichtung mechanisch und elektrisch zu verbinden.

8. Elektrostimulationsvorrichtung nach Anspruch 7, wobei der elektrische Impulsgenerator (3) mindestens zwei starre Bereiche (31, 32, 33, 34) umfasst, die durch einen flexiblen Bereich (35, 36, 37) elektrisch und mechanisch verbunden sind, wobei diese starren Bereiche Bestandteile des elektrischen Impulsgenerators ebenso wie die Anschlussmittel (30) an Elektroden aufnehmen.

9. Elektrostimulationsvorrichtung nach Anspruch 7 oder 8, umfassend Magnetmittel auf Höhe der elektrischen Anschlussmittel, die auf der kutanen Elektrodenvorrichtung (1, 6, 8) oder auf dem elektrischen Impulsgenerator (3) vorgesehen sind.

10. Elektrostimulationsvorrichtung nach einem der Ansprüche 7 bis 9, umfassend eine kutane Elektrode (5), die geeignet ist, um mit dem mindestens einen Anschlussmittel (30) des elektrischen Impulsgenerators elektrisch verbunden zu werden.

## Claims

1. A cutaneous electrode device intended to be connected to an electric pulse generator (3) and including:
a planar body (10, 60, 80) made from an electrically insulating material on which at least one electrode (11, 12; 61, 62, 63, 64; 81, 82), at least one means (13, 14; 65, 66, 67, 68; 83, 84) for connecting to the electric pulse generator (3), and at least one conductive element (15, 16; 71, 72, 73, 74; 85, 86) electrically connecting said electrode to said connecting means are formed, said at least one electrode emerging on an inner face (106, 608) of said body and said at least one means for connecting emerging on an outer face (100, 600, 805) of said body and **characterised in that** at least part (101, 601, 800) of this body has an elongate shape, extending along the longitudinal axis, and includes at least two protruding portions (102, 103; 602, 603, 604, 605; 806, 807), said at least two portions mechanically connecting an electrode (11, 12; 61, 62, 63, 64; 83, 84) and a connecting means (13, 14; 65, 66, 67, 68; 83, 84) and being intended to electrically and mechanically connect, by bending, said electrode device to said electric pulse generator.

2. The device according to claim 1, wherein the entire body is flexible.

3. The device according to claim 1 or 2, wherein said at least two portions are arranged on either side of said longitudinal axis.

4. The device according to claim 1 or 3, wherein said body (8) includes at least one branch (801, 802) extending from the elongate part of said body while forming a non-zero angle with its longitudinal axis, an electrode (81, 82) being made on said at least one branch.

5. The device according to one of claims 1 to 4, comprising a grate (17, 18; 75, 76, 77, 78) formed on the inner face of said body (10, 60) and electrically connected to said at least one electrode (11, 12; 61, 62, 63, 64).

6. The device according to one of claims 1 to 5, comprising at least one protruding portion (904, 905) complementary with a hole (906, 907), this portion extending substantially along the same axis as said at least one portion (102, 103) and on the opposite side relative to said body (10).

7. An electrostimulation device formed by a cutaneous electrode device (1, 6, 8) according to one of claims 1 to 6 and an electric pulse generator (3) including at least one connecting means (30) on its upper face (300), the lower face (302) of said electric pulse generator (3) being able to come into contact with the outer face (100, 600) of said electrode (1, 6), and at least one protruding portion (102, 103; 602, 603, 604, 605; 806, 807) being capable of coming into contact with the upper face (300) of the electric pulse generator by bending, and mechanically and electrically connecting it to said electrode device.

8. The electrostimulation device according to claim 7, wherein said electric pulse generator (3) comprises at least two rigid zones (31, 32, 33, 34) electrically and mechanically connected by a flexible zone (35, 36, 37), these rigid zones bearing the components of the electric pulse generator as well as the means (30) for connecting to electrodes.

9. The electrostimulation device according to claim 7 or 8, including magnetic means at the electrical connecting means provided on the cutaneous electrode device (1, 6, 8) or on the electric pulse generator (3).

10. The electrostimulation device according to one of claims 7 to 9, including a cutaneous electrode (5) able to be electrically connected to said at least one connecting means (30) of the electric pulse generator.
